# EUROPEAN PATENT SPECIFICATION

(11) **EP 1 787 653 B1**
(45) Date of publication and mention of the grant of the patent: **06.06.2012**
(21) Application number: 05762122.9
(22) Date of filing: 05.07.2005
(51) Int. Cl.: A61K 38/16, C07K 14/265, A61P 35/00, A61P 31/04

(54) **Antitumoral pharmaceutical composition containing polypeptide fragments of serralysins**
Antitumorale pharmazeutische Zusammensetzung mit Polypeptide-Fragmenten von Serralysinen
Composition pharmaceutique antitumorale contenant des fragments polypeptidiques de serralysine

(30) Priority: 08.07.2004 CU 14704
(43) Date of publication of application: 23.05.2007
(73) Proprietor: CENTRO DE INGENIERIA GENETICA Y BIOTECNOLOGIA (CIGB), Ciudad de la Habana 10600 (CU)
(72) Inventor: ABRAHANTES PEREZ, María del Carmen, Calle 186, Ciudad de la Habana 10600 (CU); REYES GONZÁLEZ, Jesús, Calle 186, Ciudad de La Habana 10600 (CU); VELIZ RÍOS, Gloria, Calle 140, Ciudad de La Habana 11500 (CU); MARTÍNEZ DÍAZ, Eduardo, Calle 186, Ciudad de La Habana 10600 (CU); GASMURI GONZÁLEZ, Caridad Anais, Ciudad de La Habana 11300 (CU); GARCÍA SUÁREZ, José, Calle 186, Ciudad deLa Habana 10600 (CU); BEQUET ROMERO, Mónica, Ciudad de La Habana 10400 (CU); GONZÁLEZ LÓPEZ, Luis Javier, Calle 186, Ciudad de La Habana 10600 (CU); CASTELLANOS SERRA, Lila Rosa, Kohly 51., Ciudad de La Habana 10600 (CU); SELMAN-HOUSEIN SOSA, Manuel, Ciudad de La Habana 10600 (CU); GÓMEZ RIERA, Raúl, Ciudad de La Habana 11500 (CU); GAVILONDO COWLEY, Jorge Victor, Calle G, Ciudad de La Habana 10400 (CU)
(74) Representative: Jansen, Cornelis Marinus
(86) International application number: PCT/CU2005/000003
(87) International publication number: WO 2006/005268

(56) References cited:
- EP-A- 0 226 800
- WO-A-99/15690
- FR-A- 2 519 021
- US-A1- 2004 185 566
- MONTANER B ET AL.: "Prodigiosin from the supernatant of Serratia marcescens induces apoptosis in haematopoietic cancer cell lines", BRITISH JOURNAL OF PHARMACOLOGY, vol. 3, no. 131, October 2000 (2000-10), pages 585-593, XP001091105,
- ABRAHANTES-PEREZ M C ET AL.,: "Cytotoxic proteins combined with prodigiosin obtained from Serratia marcescens have both broad and selective cytotoxic activity on tumor cells", JOURNAL OF CHEMOTHERAPY, vol. 2, no. 18, April 2006 (2006-04), pages 172-181, XP008111361,

## Description

### Field of the invention

The present invention is related to the field of biotechnology, the pharmaceutical industry and in particular with the production of a composition capable of inhibit the growth of tumoral cells. This composition contains polypeptides fragments of Serralysins, obtained from the degradation of the intact protein, which have a higher anti-proliferative activity than the one of the whole serralysin molecules. Said fragments belong to the C-terminal of the serralysins, from the internal methionine of the serralysin sequence to the end of the molecule, and the combination of the same with prodigiosins enhancing the anti-tumoral effect of this composition.

### Prior art

Cancer chemotherapy has been traditionally directed to the inhibition of cancer cells proliferation. Nevertheless, in the last years the interest in anti-tumoral products that induce apoptosis, has increased because cancer has been establish as a pathology related to a relative deficiency in apoptosis, instead of an excess of proliferation.

Bacteria or their extracts have been used for cancer treatment for almost 100 years. The most quoted report is from the physician and surgeon William B. Coley from the Memorial Hospital in New Cork city called Sloan-Kettering Memorial Hospital. He observed that many of his patient with several types of cancer experience tumor regression after been infected by pathogenic bacteria. (Coley, W. B. 1991-reprinted from 1893-. Clin. Orthop. 262:3).

Resistance to tumors in patients or animals infected was attributed to the concomitant cell mediated antitumoral immunity (Paglia, P. and Guzmán, C. A. 1998. Cancer immunol. Immunother. 46:88). The idea that a pathogenic bacterial or protozoan infection could activate cancer regression through the activation of the innate or adaptative immunity has been recently questioned by Hunter et al. (Hunter, C. A., Yu, D., Gee, M., Ngo, C. V., Sevignani, C., Goldschmidt, M., Golovkina, T. V., Evans, S., Lee, W. F. and Thomas-Tekhonenko, A. 2001. J. Immunol. 166:5878). They demonstrated that the tissues infected by T. Gondii produce some soluble antiangiogenic factors that avoid the formation of blood vessels in the tumors, which could be of potential therapeutic interest. This process of formation of new capillaries known as angiogenesis has become an important focus of attention for the implementation of new therapies for cancer and their metastases. The search for antiangiogenic factors is the foundation of new anticancer therapeutic strategies (Folkman, J. 2003. Seminars in Cancer Biology. 13:159). Recently the use of anaerobic bacteria as chemotherapeutic and selective anti-vascular agents has resulted in a significantly regression of subcutaneous (sc) tumors in mice. This treatment is named combined bacteriolytic therapy (COBALTO) (Dang, L. H., Bettegowda, C., Huso, D.L., Klnzler, K.W. and Vogelstein, B. 2001. Proc Natl Arad Sci USA. 98: 15155). Nevertheless, live bacteria produce important toxicity and collateral reactions that limit their use against human cancer.

In the last years, some reports have appeared that indicate that anaerobic bacterias release redox proteins that induce tumor cells apoptosis (Yamada, T., Goto, M., Punj, V., Zaborina, O. and Chen, M.L. 2002. Proc. Natl. Acad. Sci. USA. 99: 14088; Goto, M., Yamada, T., Kimbara, K., Horner, J., Newcomb, M., Gupta, T.K. and Chakrabarty, A. M. 2003. Mol Microbiol. 47:549). It has been postulated that redox proteins could be included in a group of soluble proteins that were secreted by prokaryotic cells ancestors, and their functions could have been the elimination of ancestral eukaryotic cells (Punj, V. and Chakrabarty, A. M. 2003. Cellular Microbiology. 5:225). In general little is known about the production of soluble secreted factors by these prokaryotic organisms that could act in a specific manner on cancer cells, causing their death and concomitantly, the tumor regression.

Serratia marcescens is a facultative anaerobic bacteria. From some of its strains some preparations have been obtained with antitumoral properties, the most studied are: [a] ImuVert^{®} (Budagov, R.S. and Ulianova, L.P. 2001. Radiats Biol Radioecol Russian. 41:38), a preparation of ribosomal membranes that activate the patient's immune system; [b] the Serratia protease Mr 56,000 (Wu, J., Akaike, T., Hayashida, K., Okamoto, T., Okuyama, A. and Maeda, H. 2001. Jpn. J. Cancer Res. 92:439), which induce the cells death by necrosis depending on the expression of α-2 macroglobuline; and [c] the prodigiosins, a family of pigments that act as immunesupressors and anti-cancerigens through the induction of apoptosis (Montaner, B and Pérez Tomas, R. 2003. Curr Cancer Drug Targets. 3:57; Pérez Tomas, R. y Montaner, B. 2003. Histol Histopathol. 18:379).

We have obtained non-proteolytic fragments of serralysins with higher cytotoxic activity than the entire molecules. This allows combining these fragments with low doses of prodigiosine, lowering the toxicity reported for the pigment and increasing the anti-proliferative effect on tumoral cells.

### Detailed description of the invention.

The compositions of this invention are able to inhibit the growth of tumoral cells and are formed by polypeptide fragments of serralysins, with higher anti-proliferative effect than the entire serralysins molecules, which can be combined with prodigiosins that enhance their anti-tumor effect.

This invention describes the composition of the MG2327 preparation, where coexist both polypeptides and prodigiosins, which have a wide spectrum of cytotoxic activity on malignant cell lines, with selective effect on transformed tumoral cells and specifically on cells activated for their growth. The sensibility study on different cell lines, tumoral or not, demonstrate that normal cell lines are only slightly sensitive to the MG2327 preparation, while cells derived from melanoma, laryngeal carcinoma, fibrosarcoma, hepatocarcinoma and cervical-uterine carcinoma (positive or not for human papilloma virus) are very sensitive. The carcinomas of hematopoietic origin are less sensitive. HUVEC cells activated for their growth are more sensitive to MG2327 that those no activated. The MG2327 preparation is able to act specifically on factors expressed or over expressed during the process of cell division. These factors constitute therapeutic targets against cancer or other diseases of proliferative origins. Furthermore, these factors also constitute targets for the early diagnosis of diseases originated by an excess of proliferation, or by the non controlled proliferation of differentiated or non-differentiated cells. Controlled release formulations containing these molecules could be directed to these proliferating targets acting in a specific manner upon them; because normal cells are more resistant to its action.

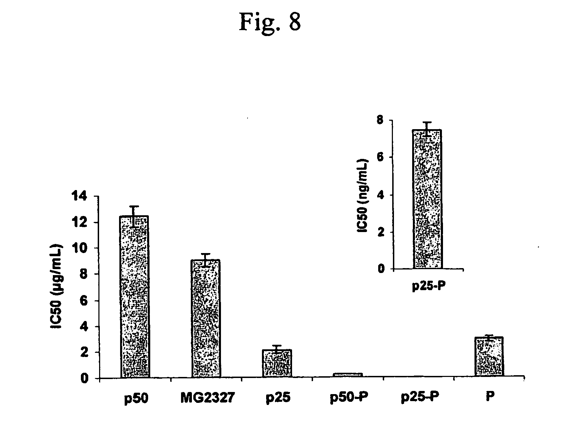

In order to demonstrate the anti-tumor activity of the MG2327 preparation BALB/c mice were challenged with an intraperitoneal inoculation of tumor cells CB Hep.1 of myeloid origin able to cause ascitic murine tumors (Fontirrochi, G., Dueñas, M., Fernandez de Cossio, M.E., Fuentes, P., Pérez, M., Mainet, D., Ayala, M., Gavilondo, J.V. and Duarte, C.1993. Biotecnol Aplic. 10: 24-30). After 10 days, mice were injected intraperitonealy with MG2327 preparation or PBS. The 60% of the animals treated with 1 mg/kg survived, while only the 25% of the controls were alive 45 days after the beginning of the treatment (Fig. 8). Total tumoral regression was observed in the treated survivors, showing a healthy condition, while in the controls, the tumors progressed forming big solid masses and mice showed a deterioration of the general condition.

BALB/c mice bearing tumors of myeloid origin treated with one dose of 1 mg/kg of weight of the MG2327 preparation survived with total regression. This same dose increases the survival with a significant reduction of tumor volume in BALB/c mice bearing a tumor originated by E6/E7 transformed fibroblast. MG2327 protect BALB/c mice from the implant of myeloid tumors.

The MG2327 preparation was obtained as a result of the optimization of the culture conditions to produce anti-proliferative molecules. MG2327 preparation was obtained as result of the optimization of the culture conditions to produce anti-proliferative molecules, which constitute a protector agent against the implant and development of malignant tumors, in this manner as inductor of the production of anti-proliferative, apoptotic and anti-angiogenic molecules in normal and tumors cells, that could be used advantageous in the prophylaxis and in the therapy of the cancer, moreover of other diseases related with this events.

CMIB 4202 strain over-express soluble proteins in the range of 45-50 and 20-30 kDa (50 and 25 kDa as determined by SDS-PAGE, with one determination coefficient of 0.984). The fraction of 25 KDa, named p25, showed a doses-dependent potent anti-proliferative activity in the experiment perform with the HEp-2 cellular line incubate with EDTA, while that the 50 KDa fraction named p50,did not inhibit the growing. However, the p50 fraction incubated with 5 µM Zn₂SO₄ showed anti-proliferative activity, but less potent than that of the p25 fraction. The IC₅₀ of the p25 and p50 fractions were 0.48 nM and 16 nM, respectively.

The isolated bio-molecules with anti-proliferative effect (polypeptides and prodigiosin) were performed in the present invention by only one chromatographic step: ionic interchange using a discontinuous gradient of NaCl. A DEAE or QAE Sepharosa Fast Flow matrix was used; equilibrated with 50 µM of phosphate buffer, pH 8.00. The elution was performed with one discontinuous gradient of NaCl: 50 mM of phosphate buffer -0.1 M NaCl, pH 8.00; 50 mM of phosphate buffer-0.2 M NaCl, pH 8.00; 50 mM -2 M NaCl, pH 8.00 and finally was the pigment fraction absorbed to the matrix was eluted with 70 % absolute ethanol. The results confirm that protein component preparation of 25 kDa have a higher capacity than the protein component of 50 kDa of the same preparation, to inhibit the growth of tumor cells, and both present in vitro biologic activity of independent form.

Fragments of several molecular sizes provoked by the degradation of p50 included fragments of 25 kDa. Increased degradation of p50 was obtained with high temperature, and the generation of p25 was proportional with the temperature increment. The anti-p50 polyclonal antibodies, obtained in sheep, recognized p25 in Western Blot assay, therefore the p25 was originated as a product of the degradation of the p50 protein. The products anti-proliferative activity increased proportionally with the degradation state. These results confirm that the p50 autolysis is able of produce degraded fragments with anti-proliferative activity more potent than the original molecule (p50). Moreover, the p25 protein also induces total regression of malign tumors of myeloid source. The fragments of p50 can be genetically conjugated with antibody fragments, by known methodology to generate inmunotoxins, useful to treat disease of proliferative etiology. Also this fragment alone or combined, with other proteic molecules may be employed as carrier. The proteolytic activity of p50 was inhibited with 7 mM of EDTA, which demonstrated that p50 is a metalloprotease, which was identified by mass spectrometery, and belong to the Serralysins family. The major similarities of our fragments were found with the species identified as PRZN_SERSP and PRZN_SERMA in the database of Swissprot proteins. Chromatographically purified p25 protein has no enzymatic activity and it correspond to the non catalytic carboxyterminal region of Serralysins.

The protein components and the prodigiosin were formulated in same composition which increased significantly (p<0.005), the inhibitory effect with respect to independent form of the formulation. This composition was obtained maintaining the same proportion of proteins as prodigiosin that the ones used when the components were evaluated independently. In such composition the prodigiosin can be found in concentration of 0.1 - 100 nM, and the Serralysin fragments between 0.1 - 150 µg/mL.

It is an object of this invention to obtain compositions for use containing polypeptide fragments derived from Serralysins with greater anti-proliferative effects than the integral Serralysins molecule and the combination of these fragments with Prodigiosins which selectively enhance the biological activity of the composition.

Additionally, these polypeptide fragments have apoptotic effect on cancer cells. This apoptotic effect involved mitochondria, microtubules and DNA fragmentation, amplifying the program cellular death signal, using low doses of these compositions. These events also were observed with combined compositions of polypeptide and prodigiosin.

Anti-angiogenic effect of MG2327 and anti-proliferative polypeptides fragments were evaluated by the method of formation of tubular structure in matrigel. Non cytotoxic concentration of MG2327 and their fraction p25 and p50 were incubated with human microvasculature endothelial cells (HMEC). The results were evaluated considering the tubular structure length formed and the number of interconnection between them, using the Pro Express 4.5 Image- program. The differentiation or maturation of endothelial cells was inhibited significantly (p<0.05, ANOVA) by MG2327 composition and its anti-proliferative polypeptides), demonstrating anti-angiogenic activity of MG2327 and its anti-proliferative polypeptides.

Apoptotis, anti-angiogenic activity and the selectivity are ones of the most important characteristics of composition according to the invention due to its potential therapeutic activity and protective effect against the cancer.

In the present invention is disclosed that the combinations of Serralysins fragments with the prodigisins are more potent and selective when they are used independently as anticancer agents. These polypeptides can be used for obtaining toxins or recombinant immunotoxins for prophylaxis and cancer therapies, or others diseases related with proliferation of endothelial and transformed cells. These polypeptides and their possible combination with prodigiosins can be used in the pharmaceutical industry to obtain vaccine preparations, therapeutic compositions or diagnostic reagents for human or animal use, against cancer and other proliferative pathologies that are highly selective and of the high spectrum of action.

### FIGURE DESCRIPTION

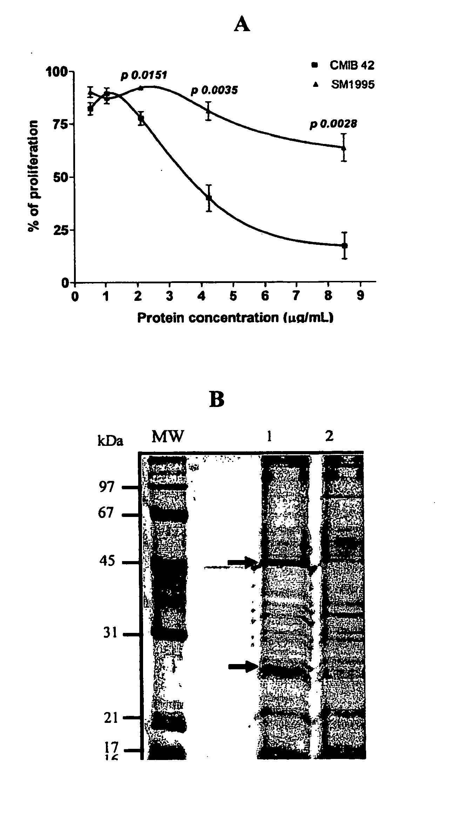

Figure 1. The interaction of *S. marcescens* with tumoral cells CB Hep.1 generates bacterial strains with high anti-proliferative capacity and with modified protein expression. A- Cell survival. After 72 hours, cell survival was estimated by the MTT method. The strain CMIB 4202 showed a strong anti-proliferative effect on the human tumoral cells HEp-2, while the effect of the SM1995 strain was very weak. These results were the average of tree independent experiments using four replicas per sample. B- SDS-PAGE electrophoresis (silver staining). CMIB 4202 over-expressed soluble proteins migrating around 25 and 50 kDa.

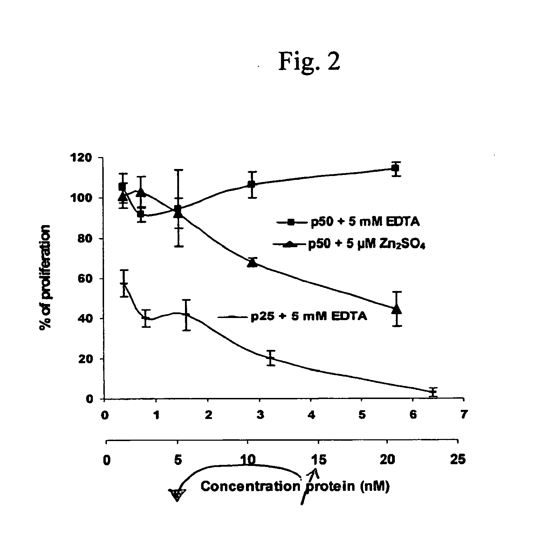

Figure 2. Anti-proliferative activity of the 25 and 50 kDa fractions on the cell line HEp-2. Cell viability was determined by the MTT method and expressed in percents with respect to the control cells. The fractions were recovered from SDS gels (stained with zinc-imidazol) and renaturalized. The fraction around 25 kDa showed a strong anti-proliferative activity (IC₅₀ 0.35 nM/mL), while p50 was unable to inhibit the growth. When Zn₄SO₂ 5 µM was added to the p50, an increase in the antiproliferative activity was observed (IC₅₀ 45 nM/mL) although inferior to that of the p25. The curves were generated from the mean values of five independent experiments and are plotted with the correspondent standard deviation (SD).

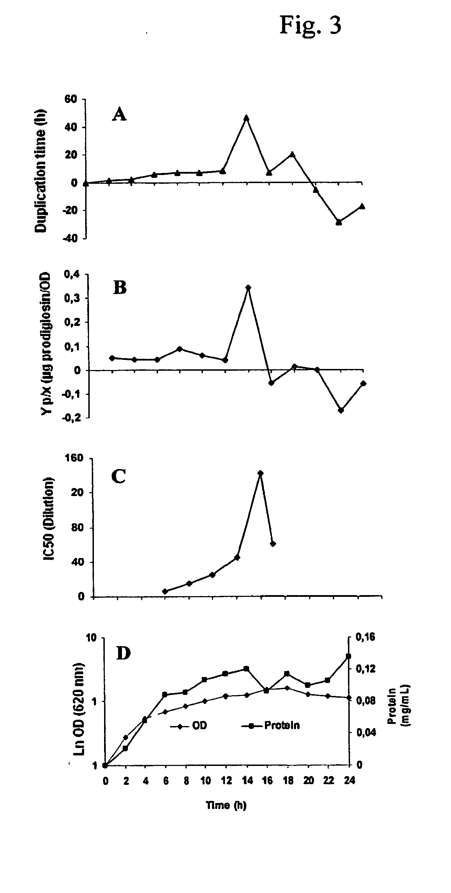

Figure 3. Kinetics of protein and prodigiosine expression by the strain CBMI 4202. The expression of prodigiosins to the culture media occurs during the transition period from the growing phase to the stationary phase, where CBMI 4202 reach its higher duplication time. A- Kinetics of the cell duplication time.
B- Efficiency of the prodigiosine expression (product/biomass). C- Kinetics of the anti-proliferative effect on Hep-2 cells, determined by the MTT method. D-Kinetics of the cellular growth determined by optical density and protein biosynthesis.

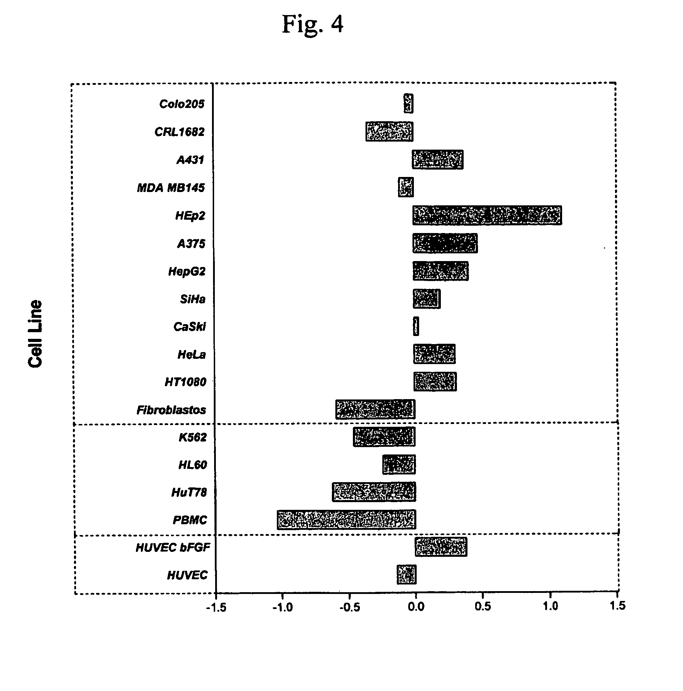

Figure 4. Sensitivity of tumoral and normal human cells to the treatment with MG2327. The normal cells have low sensitivity and those of haematopoietic origin are less sensitive than the rest of the analyzed cell lines. Meanwhile cells activated for growing (HUVEC bFGF) and cells derived from tumoral malignant lesions are more sensitive.

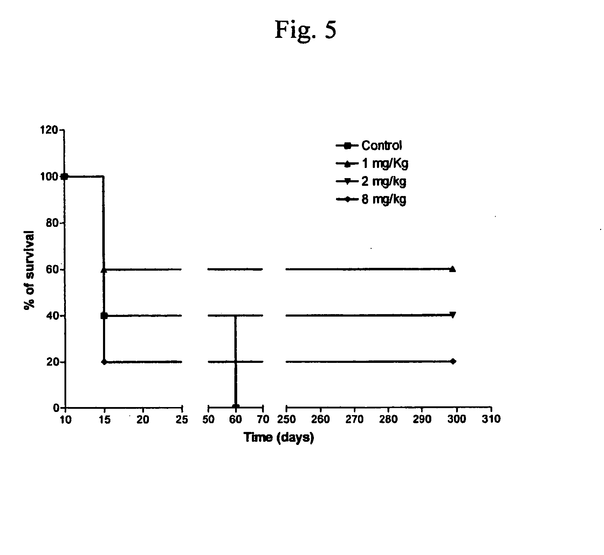

Figure 5. Analysis of BALB/c mice survival after been treated or not with MG2327 preparation and challenged with the CB-Hep.1 tumor. A- The dose of 1 mg/kg induced a tumor regression in the 60% of the treated animals, while 100% of nontreated animals died by day 60. B- Forty five days after tumor cells inoculation, non-treated animals showed an extremely weaken status, with the presence of solid tumors and ascites, while those treated with 1 mg/kg do not displayed evidences of tumors and survived for more than 300 days.

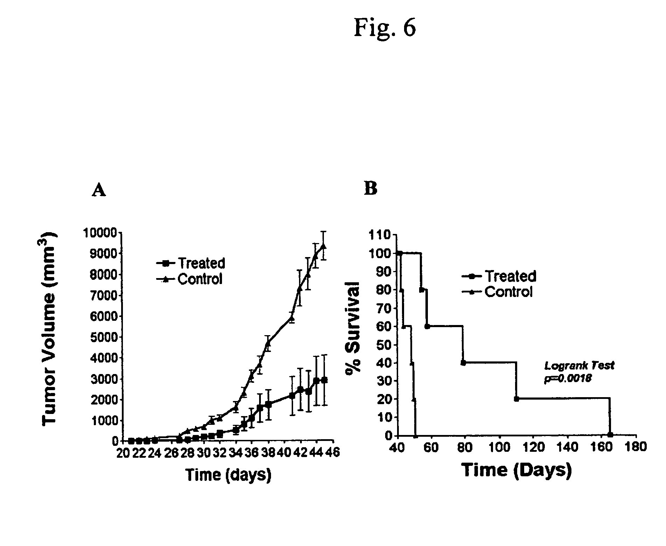

Figure 6. Antitumoral effect of MG2327 on the tumoral model 3T316. A- The plot of the day to day mean for each group, reveal statistically significant differences among the tumoral volumes of treated and non-treated animals (p<0.003). The analysis of tumor growth through the time evidences no significant variations (p= 0.109) for the group treated with MG2327, while the negative control group showed a significant increase in this parameter (p= 0.04). B- Survival post treated and non-treated animals.

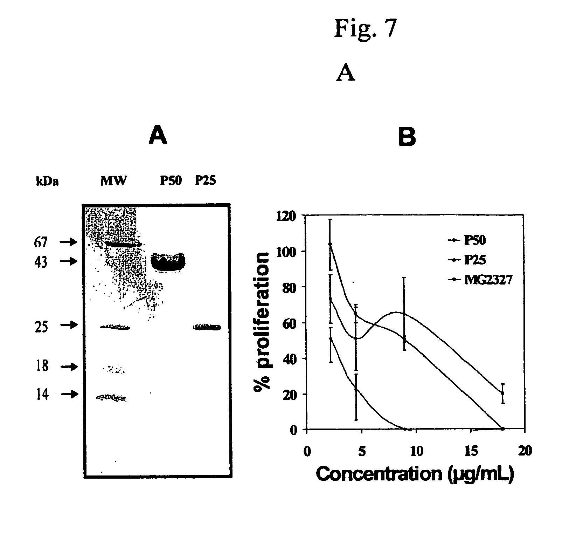

Figure 7. Isolation of the protein active principles from MG2327. (A) Electrophoresis in SDS-PAGE (12.5% polyacrylamide), revealed by Coomassie staining. Lane 1 shows the sample corresponding to the elution with 0.2 M NaCL, pH 8.00, where a protein band corresponding to 50 kDa can be appreciated. Lane 2 shows a protein band at 25 kD a. (B) Anti-proliferative effect on HEp-2 of p50 and p25 proteins on human tumoral cells. Dose-response relation to p25, p50 and MG2327 expressed in terms of total protein concentration.
Figure 8. Anti-proliferative effects of the active principles isolated from MG2327 on HEp-2 cells. The 50 and 25 kDa proteins enclosed in MG2327 showed a growth inhibition activity. The combination of these proteins with prodigiosins reduced the dose able to inhibit the growth of the 50% of the tumoral cells as compared to control (IC₅₀).

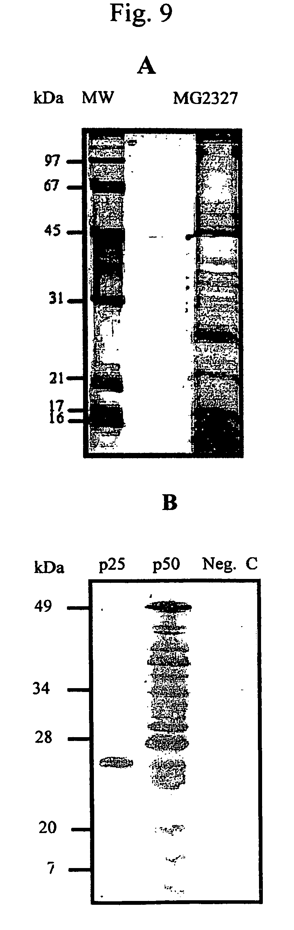

Figure 9. SDS-PAGE and Western Blot. (A) Pattern of protein obtained from MG2327 using SDS-PAGE (12%) gel (silver stained). The protein bands of approximately 50 and 25 kDa (arrows) were cut from a similar gel stained with a zinc-imidazole, renaturalized and re-run in a new SDS-PAGE. This was transferred to a nitrocellulose membrane and the western blot was developed with an anti-p50 polyclonal antibody obtained in goat. The polyclonal antibodies were able to recognize the p25 and the degradations of the p50 and do not recognize the non related protein bands (Neg C).

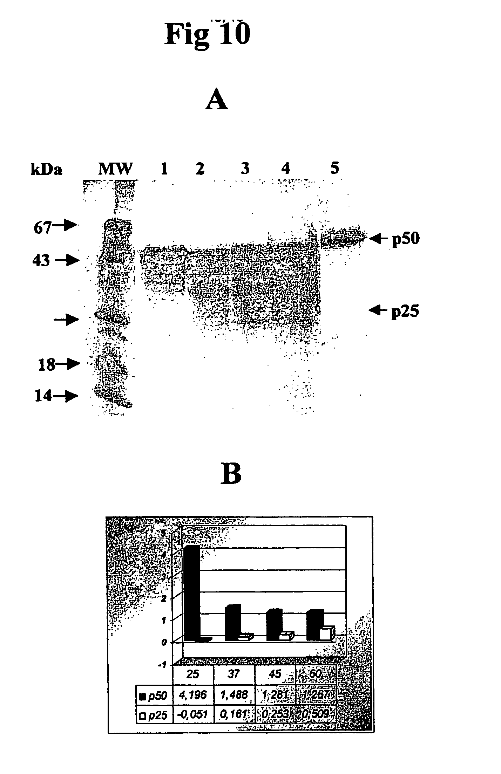

Figure 10. p50 autolysis. A- SDS PAGE electrophoresis: 1- 24 °C, 2- 37 °C, 3- 45 °C, 4- 60 °C, 5- 4 °C. B- The densitometric analysis of the p50 and p25 showed that with the increase in the temperature of incubation the intensity of the p50 band is decreased while the intensity of p25 band is increased.

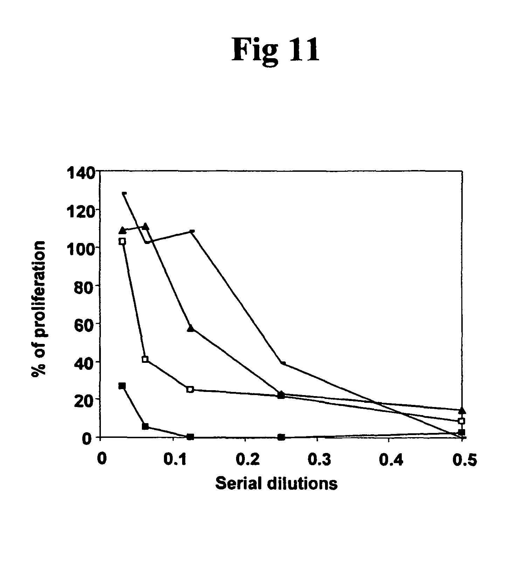

Figure 11. p50 purified by DEAE Sepharosa Fast Flow chromatography. The p50 (eluted with 0.2 M NaCl), generated degradations with higher anti-proliferative activity than the parental molecule.

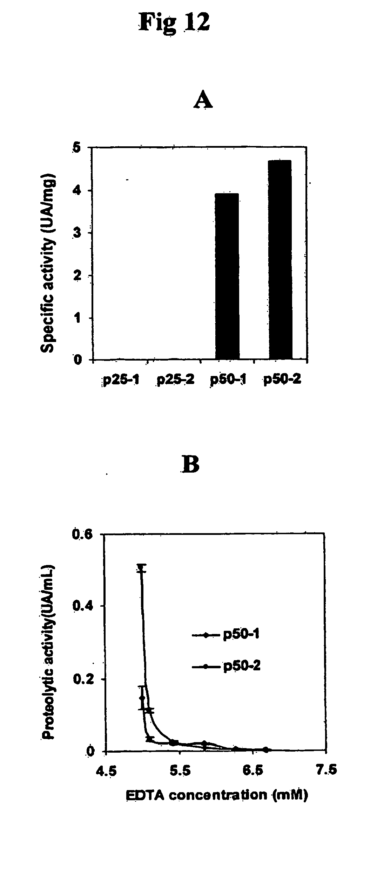

Figure 12. Enzymatic activity of p25 and p50 obtained from different chromatographies. A- p25 does not present activity, while p50 showed enzymatic activity. B- The enzymatic activity of p50 was totally inhibited with 7 mM of EDTA.

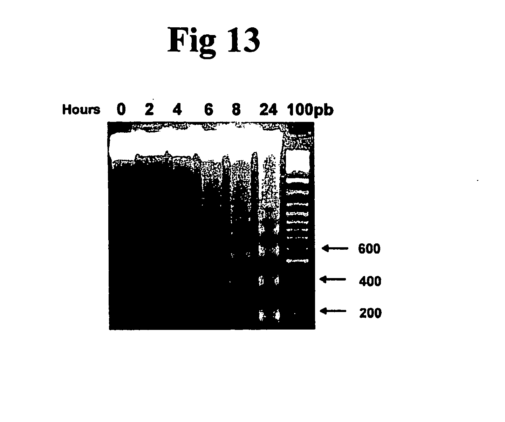

Figure 13. MG2327 induced the time-dependent DNA fragmentation of the tumoral cells P3X63Ag8. Since the 6 hours of incubation the oligonucleosomal fragments can be observed and they increased with time, reaching the typical apoptotic pattern with oligonucleosomal fragments of 180-200 base pairs at 24 hours.

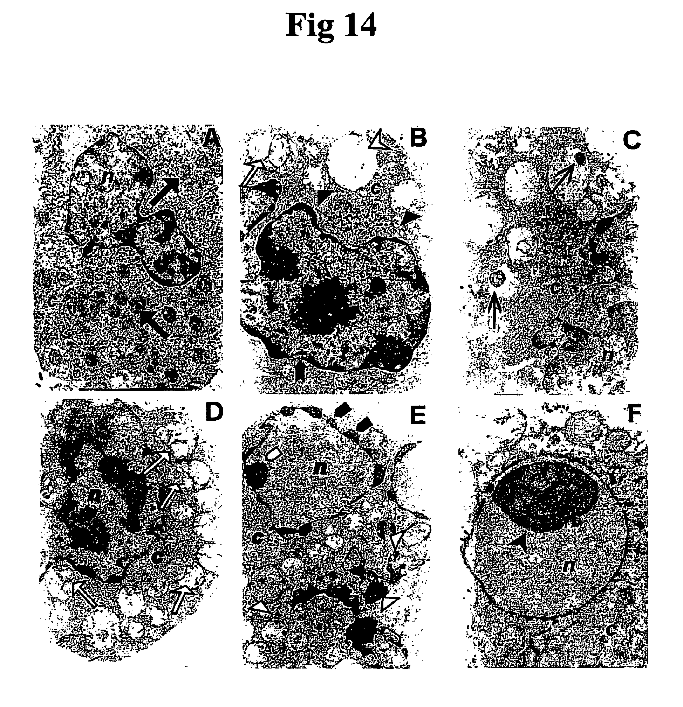

Figure 14. Fig. 2 Ultrastructure of P3X63AG8 murine myeloma cells treated and untreated (control cells) with MG2327 (MG) preparation at 22 µg/ml. (A) Electron micrograph of control P3X63Ag8 myeloma murine cells showed cytoplasm and nucleus of theses cells showed ultrastructure typical for normal cells and mitochondrial ultrastructure typical for normal cells: mitochondrial matrix had a higher density than the surrounding cytoplasm ( 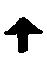 ). (B) Vacuolazed bodies (which in part derive from altered mitochondria ( 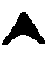 )), mitochondrial swelling and disruption of cristae were present in the cytoplasm (▲), with normal nucleus ( 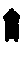 ) at 2 h after MG treatment. (C, D) Mitochondrial clustering and individual cristae become fused ( 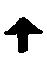 ), also observed after 2 hours of treatment in (B). (E) Electron micrographs with apoptotic morphology: condensation ( 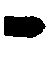 ), margination and fragmentation of chromatin ( 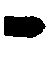 ), and apoptotic bodies (Δ) at 6 h after MG2327 treatment. (F) Apoptosis revealed by compacted chromatin, the nucleus showed peripheral patches of condensed chromatin. Note mitochondrial swelling and disruption of cristae, whole cytoplasm membranes were present on all time different. Magnifications: x6000 (E), x 10 000 (A,B,D), 15 000 (C) and x40 000 (F).

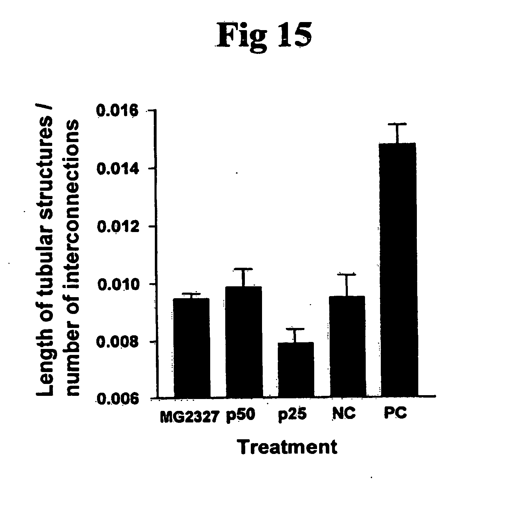

Figure 15. Effect of MG2327, p25 and p50 (purified by chromatography) on the differentiation of endothelial cells in Matrigel. HMEC cells were cultured in activation conditions (10 ng/mL EGF, 1 µg/mL of hydrocortisone) in the presence of similar concentrations of MG2327 (A), p50 (B) and p25 (C), no treatment (E) and without activation (D). In the graph (F) the results of 3 independent experiments are grouped, showing the inhibitory activity of MG2327 and its components on the formation of tubular nets in matrigel. Both MG2327 and p50 completely revert the activation induced to the level of non-activated cells (ANOVA MG2327, p50 and CN p>0.05). The cells treated with p25 showed an index of net formation inferior to that observed for MG2327 and p50 (unpaired t p=0.0107 and p=0.0498, respectively).

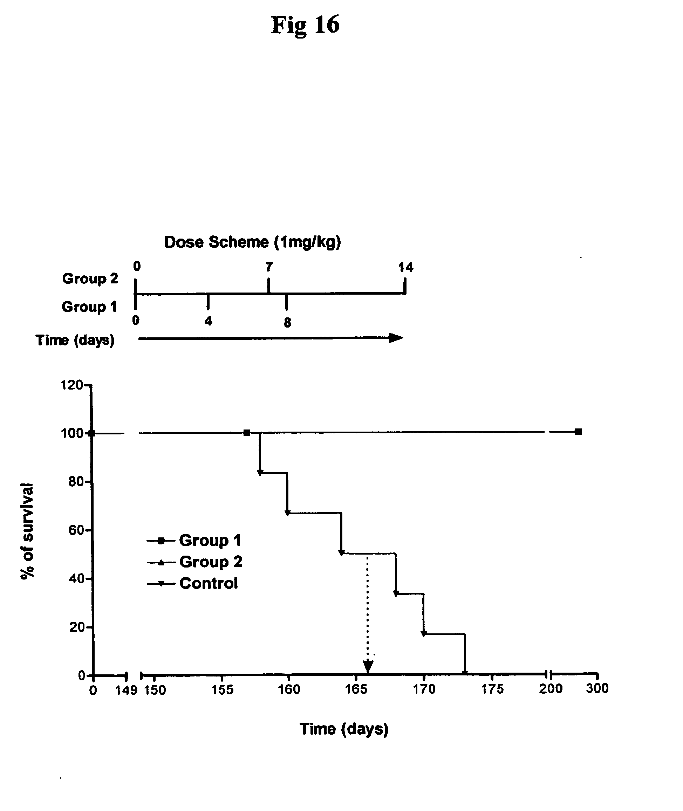

Figure 16. Survival of BALB/c immunized or not with MG2327 and challenged with X63 myeloma cells. Using 3 and 6 doses of 1 mg/kg of MG2327 the 100% of the immunized animals reject the myeloid tumor.

### EXAMPLES

### Example 1. Obtaining the strain CMIB4202.

In order to obtain bacterial strains producers of antitumoral molecules, the wild type *Serratia marcescens* SM1995 isolated from the ventral surface of BALB/c mice was mixed with the tumoral cells CB-Hep.1 (Alemán, M.R., Valdés, R., Pérez, M., Ibarra, N., Reyes, B., González, M., Mendoza, O., Padilla, S., Agráz, A. and Rodriguez, M. P. 2000. Biopharm 13:48-52) and were inoculated intraperitoneal in BALB/c mice, previously inoculated 10 days before with heavy liquid petrolate. Eight days after the inoculation of the cells mixtures, ascitic fluid extractions were performed every 2 days. The kinetics of tumor growth was analyzed and the microbiological control was performed to the ascites of each animal presenting tumor regression.

The isolated bacteria were grown in different media and culture conditions. The culture supernatants were sterile filtered using membrane filters of 0.22µm and their toxicity was evaluated on CB-Hep.1 cells. The strain with higher cytotoxicity was deposited with the accession number CMIB4202 in the Collection of Microorganism with Biotechnological Importance of the Center of Genetic Engineering and Biotechnology, Habana city, Cuba. CMIB4202 and its parental strain SM1995, were cultured in parallel in 5L fermenter, in peptone-glycerol media at 28°C. The sterile filtrate of CMIB4202 evidence a dose dependent activity while the one from SM1995 has only a little activity on the human cancer cell line HEp-2 (Fig.1A) in the anti-proliferative assay using the MTT method (Skehan, P., Storeng, R., Scudiero, D., Monks, A., Mcmahon, J., Vistica, D., Warren, J. T., Bokesch, H., Kenney, S. and Boyd, M. R. 1990. J. Natl. Cancer. Inst. 82:1107).

The strain CMIB4202 over-expressed soluble proteins in the ranges of 45-50 and 20-30 kDa (50 and 25 kDa as determined by SDS-PAGE, with a coefficient of determination of 0.984), Fig. 1B.

The p25 fraction recovered from the bands presented in SDS gels stained with zinc-imidazole (Hardy, E., Santana, H., Sosa, A., Hernández, L., Fernández-Padrón, C. and Castellanos-Serra, L. 1996. Analytical Biochemistry. 240:150) showed a strong antiproliferative dose dependent activity in HEp-2, while the p50 fraction does not inhibit the cell growth. Nevertheless, when p50 was incubated with 5 µM Zn₂SO₄ showed anti-proliferative activity but lower as the observed for p25 (Fig. 2). The IC₅₀ of the fractions p25 and p50 were 0.48 nM and 16 nM, respectively.

With the objective of compare the ability of both strains to express the proteins, a factorial ANOVA was applied. The value of the probability of interaction was not significant (p= 0.93). On the other hand, the probability of both main effects showed that both proteins are expressed in different amount significantly (P =0.01) and that this amount is highly strain depended (P=0.0004). Furthermore, there were significant differences in expression for these proteins between both strains (P<0.001).

### EXAMPLE 2. Obtaining the MG2327 anti-proliferative preparation.

For obtaining one anti-proliferative preparation, from the CMIB4202 *S. marcensces* strain, 1L of culture of microorganism and media of culture optimal was made to produce molecules of interest (Fig. 3). The CMIB4202 culture was centrifuged to 12 000 g and 4 °C by 30 minutes. The supernatant was collected and subsequently filtrated by molecular cut-off of 0.2 µm under sterile conditions. The volume of the supernatant was reduced 10 times in the same conditions of sterility. The volume of the supernatant was 10 times reduced using a membrane of 10 kDa exclusion limit and dialyzed against PBS to 4 °C during 24 h, physiological conditions. Material dialyzed was filtrated in sterile conditions, and was dispensed in apirogenics crystal vials of 5 mL. The preparation was stored at 4 °C and named MG2327. Scaling to fermenters of 5L was realized with conditions already established on screen: peptone-glycerol media to 28 °C by 14 h, aeration 1 vvm, 250 rpm y 0.1 of the initial optical density. The media culture was adjusted to physiological pH, and the fermentation was made in neutral free pH. The rest steps were done in the same form as in the shaker.

### EXAMPLE 3. Characterization of the anti-proliferative activity of the MG2327 preparation "in vitro".

For the characterization of the anti-proliferative activity of MG2327 "*in vitro*" a panel of human cell lines was evaluated (table 1). A total of 2000 cells, except for PBMC (20000), were seeded in 96 wells culture wells, and different concentrations of MG2327 were added. After 72 hours, the number of surviving cells was estimated by addition of MTT (Skehan, P., Storeng, R., Scudiero, D., Monks, A., Mcmahon, J., Vistica, D., Warren, J. T., Bokesch, H., Kenney, S. and BOYD, M. R. 1990. J Natl Cancer Inst. 82:1107). The soluble formazan products were detected at 540 nm in a multiscan plate reader. MG2327 showed a wide range of cytotoxic activity against the analyzed human cell lines. The IC50 was in the µg/mL range

**Table 1. Cells and culture media used on the "in vitro" study**

| *FIBHUM* | *HUMAN FORESKIN DERIVED FIBROBLAST, P 3-6* | *DMEM 15% FBS*, INSULIN 30 µG*/*ML* |
|---|---|---|
| *H82* | *Human lung carcinoma* | *RPMI 1640, 10% FBS* |
| *MDA-MB145S* | *Breast human adenocarcinoma* | *DMEM, 10% FBS* |
| *Colo-205* | *Human colon carcinoma* | *RPMI 1640, 10% FBS* |
| *HT1080* | *Human fibrosarcoma* | *DMEM, 10% FBS* |
| *Hela* | *Human cervical carcinoma* | *DMEM, 10% FBS* |
| *HEp-2* | *Human laryngeal carcinoma* | *MEM, 10% FBS* |
| *HepG2* | *Human hepatocarcinoma* | *DMEM, 10% FBS* |
| *A375* | *Human melanoma* | *DMEM, 10% FBS* |
| *PBMC* | *Human Peripheral mononuclear cells* | *RPMI 1640, 10% FBS, IL-2* |
| *HuT78* | *Human Cutaneous T cell lymphoma* | *RPMI 1640, 10% FBS* |
| *HL60* | *Human promyelocylic leukemya* | *RPMI 1640, 10% FBS* |
| *K562* | *Human eritroleukemya* | *RPMI 1640, 10% FBS* |
| *CRL 1682* | *Human pancreas adenocarcinoma* | *RPMI 1640, 10% FBS* |
| *A 431* | *Human vulvar adenocarcinoma* | *RPMI 1640, 10% FBS* |
| *SiHa* | *Human cervical carcinoma* | *RPMI 1640, 10% FBS* |
| *CaSki* | *Human cervical carcinoma* | *RPMI 1640, 10% FBS* |
| *HUVEC* | *Human vascular endothelial cells* | *M199 30% FBS 10 ng*/*mL bFGF* |

| | | |
|---|---|---|
| *FBS: Fetal Bovine Serum | | |

MG2327 selectivity was compared to the commercial drug Doxorubicin (DXR) using the HT1080 cell line (from a fibrosarcoma) and primary fibroblasts. The anti-proliferative assay used was described above. Serial dilutions of DXR and of the MG2327 preparation were made starting from 10 µg/mL and a five point curve was generated. The mortality ratio was calculated for each point as the relation between the mortality percent for HT1080 and the mortality percent for the primary fibroblasts. The higher differences were detected at the lower concentrations tested (MG2327 9:1, DXR 1.7:1). MG2327 was very selective at concentrations below 2 µg/mL.

HEp-2 cells originated from a laryngeal carcinoma are very resistance to the anti-tumoral used in clinic as compared to the other cell lines analyzed. For this reason, we used it as a model for the "*in vitro*" studies of the effects of the MG2327 preparation. A comparison of the cytotoxicity curves generated for HEp-2 cells treated with known antitumoral drugs, showed similar results (40 %) of proliferation at 3 µg/mL for MG2327 preparation, Cisplatin (CDDP), Doxorubicin (DXR), Vincristine (VC), Vinblastine (VB) and Taxol (TX) (p> 0.05, test de ANOVA). In the same conditions other anti-tumoral like Ara C, Metotrexate (MTC), Bleomicine (Bleo) and Ciclophosfamide (CPA), did not showed effect. CDDP is one of the anti-tumoral authorized by the FDA for the treatment of laryngeal cancer, and the analysis of the survival curves for MG2327 preparation and CDDP showed similar values for the IC10, IC₅₀ and IC₉₀

The sensitivity study of the different cancer cell lines or non cancer cells (Fig. 4) showed that normal cellular lines have lower sensitivity while that of melanoma, laryngeal carcinoma, fibrosarcoma, hepatocarcinoma, and Cervical-uterine carcinoma (carrier of the human papilloma virus ), are very sensitive. The carcinomas of the hematopoietic origin are less sensitive. The HUVEC cells activated to grow are more sensitive to the MG2327 preparation than the ones not activated.

The previous results showed that the preparation MG2327 has a broad spectrum of cytotoxic action over malignant cells lines, with selective effect on tumors/transformed and activate growing cells.

### EXAMPLE 4 Anti-tumor activity of the MG2327 preparation.

To demonstrate the anti-tumoral activity of the MG2327 preparation we employed BALB/c mice, that were implanted intraperitoneally (i.p.) with CB-Hep.1 tumor cells, of myeloid origin, able to give rise of murine ascitic tumors (Fontirrochi, G., Dueñas, M., Fernández de Cossio, M.E., Fuentes, P., Pérez, M., Mainet, D., Ayala, M., Gavilondo, J.V. and Duarte, C.1993. Biotecnol Aplic. 10: 24-30). After 10 days, mice were injected i.p. with MG2327 or PBS. Sixty percent of the animals treated with 1 mg/kg of weight survived, while only 25 percent of the controls lived 45 days after initiated treatment (Fig. 5). Total tumor regression was observed in all treated surviving animals, that showed a healthy state, while in the controls the tumors progressed forming large solid masses, and the animals exhibited an unhealthy general state.

BALB/c mice bearing a tumor of fibroblasts transformed with E6/E7 increased their survival after being treated with the MG2327 preparation, showing a significant reduction of tumor volume.

Also, to evaluate the anti-tumoral activity of MG2327, a model of cancer associated to the human papilloma virus (HPV16) developed by Hernández et al. (Hernández, P., Merina, N., López-Ocejo, O. and Arana, M. J. 2000. Biochem Biophys Res Commun.270:119-124) was used. Two groups of BALB/c mice were inoculated subcutaneously (s.c.) with 2x10⁶ 3T316 cells in the left ventral zone. After 48 hours, a dose of 0.75 mg/kg of weight of MG2327 or PBS was administered s.c., near the primary cell inoculation in the control group, daily measurements were done with a caliper. The tumor volume was calculated using the standard form V= 0.52 x a² x b, where a is width and "b" is the length of the horizontal perimeter of the tumor (Hernández, P., Merina, N., lópez-Ocejo, O. and Arana, M. J. 2000. Biochem Biophys Res Commun.270:119-124). Behavior is shown in Fig. 6.

The differences in the time of tumor development were statically significant (p=0.0054) between the treated and non-treated animals. The study of the relationship time: treatment with the ANOVA test showed that the same magnitude of difference doesn't maintain between the treated and non treated groups; this indicated the existence of a difference related to the treatment applied to the animals.

When a Wilcoxon test was applied for the paired data to the measurements between days 21 and 45 of each group, we detected a significant increase of tumor volume for the control group (p=0.043), not being this the case for the treated group (Fig. 6A). To analyze the existence of significant differences between the groups in each moment of evaluation, we applied a Mann-Whitney U test that detected significant differences, exception made of the first point (p<0.01). Also, we detected an important difference in the speed of tumor growth. The growth curves were adjusted to one line, and the slopes were calculated from the equation generated by the best fit. The slope comparison indicated that the tumor in the control group grew at a speed significantly higher that the observed for the treated group (p=0.0088).

The mice of the control group died between 45 and 64 days due to the tumor implantation, while the animals in the treated group started to die in day 52, with a 20% survival that maintained in time (170 days), Fig. 6B.

Adjusting the survival data to a Bayesian hierarchy model (Weibull regression with 500 iterations) we obtained a statistically significant difference (p= 0.02447), that was confirmed when it was shown that the confidence intervals for the average survival time were totally exclusive.

### EXAMPLE 5. Fractions of the MG2327 preparation. Isolated of the non protein biomolecules.

Separation of MG2327 fractions was performed to determine its composition, and fractions were evaluated for the capacity to inhibit the cellular growth of the human tumor cellular line Hep-2.

The polysaccharide fraction (tr = 6.85 min) was separated in Aminex gel HPX 87-N chromatography (dimensions: 300 x 7.8 mm, flow: 0.5 ml/min).Was utilized patterns of fructose tr = 13.15 min., glucose tr = 12.12 min., disaccharide tr = 9.40 min., trisaccharide tr = 8.24 min., polysaccharide tr = 7.01 min. The pigment fraction was separated in a butyl TSK column of the MERCK equilibrated with phosphate 20 mM, pH=7, where remain retained into the matrix, subsequently was elute employing absolute ethanol. Absorption spectrum (ethanol 100 % a pH 5.00) of the product obtained showed a band with maximum 470 y 490 nm and a maximum peak in 537 nm, that correspond with characteristic describe of the monomers and dimer of the, respectively, with reported anti-proliferative activity (Pérez-Tomas, R. and Montaner, B. 2003 Histol. Histopathol. 18: 379-385; Montaner, B., and Pérez Thomas, R. 2003. Curr Cancer Drug Targets. 3:57-65). The isolated polysaccharide showed non inhibitory effect, while that fraction corresponding to prodigiosin showed dose-dependent anti-proliferative activity.

### EXAMPLE 6. Fractions of the MG2327 preparation. Isolation of the protein biomolecules with anti-proliferative effect, mediate only one chromatography step: ionic interchange with NaCl discontinuous gradient.

MG2327 preparation was applied to a *DEAE Sepharose Fast Flow* matrix; equilibrate with 50 mM of buffer phosphate, pH 8.00. The elution was perform with a NaCl discontinuous gradient: 50 mM of buffer phosphate-0.1 M NaCl, pH 8.00; 50 mM of buffer phosphate-0.2 M NaCl, pH 8.00; 50 mM of buffer phosphate-2 M NaCl, pH 8.00 and finally the pigment fraction absorbed to the matrix was eluted with 70%.absolute ethanol.

The fraction corresponds to 0.2 M NaCl, pH 8.00 and the first collected eluate of the no stick fraction (named the pass), showed dose-dependent anti-proliferative activity in the already described test. SDS- PAGE electrophoresis was observed a protein band to height of 50 kDa and a maximum band (purity > 90 %) to height of 25 kDa, respectively (Fig. 7A).The molecular weight were calculated mediated the function that relate the molecular weight of the commercial pattern with the distance of migration of the bands; r²=0.984.

The figure 7B. Show the anti-proliferative effect of p50 and p25 compared with the MG2327 preparation. p50 and p25 showed anti-proliferative effect on HEp.2 cell line.

The Table 2 shows the compared results between p50, p25 and the MG2327 preparation, employing the statistical analysis of variance (ANOVA). It compares the response of every employed dose. It is possible to observe significant differences between the activities of the three analyzed sample. These differences depend of the employed doses. To high concentration of the preparation components (9 and 18 µg/mL) significant differences exist between the fraction of 25 and 50 kDa, where the fraction of 25 kDa was more active, not being in this manner to lower concentrations (2.25 and 4.5 µg/mL).

**Table 2. Results comparatives between the protein components and the MG2327 preparation, employing the statistical analysis of variance (ANOVA).**

| **Concentration (µg/mL)** | **ANOVA** | | | |
|---|---|---|---|---|
| | **Inter-samples** | **25-50** | **25-MG2327** | **50-MG2327** |
| 2.25 | 0.0110 | 0.0649 | 0.0089 | 0.0958 |
| 4.5 | 0.0316 | 0.0685 | 0.0081 | 0.3728 |
| 9 | 0.0040 | 0.0318 | 0.0151 | 0.4271 |
| 18 | 0.0015 | 0.0243 | 0.3206 | 0.0243 |

Between the protein component of de 50 kDa and the MG2327 preparation a-significant difference exists to the dose of 18 µg/mL where the preparation was most active, obtaining 100% growth inhibition of the tumor cells, while the protein component of 50 kDa could inhibit approximately 80 % of the growth. To the doses of 2.25, 4.5, and 9 µg/mL no significant difference existed between the response provoked by the fraction of 50 kDa and the MG2327 preparation.

However, the activity of the protein component of 25 kDa was significantly different from the MG2327 preparation at doses of 2.5, 4.5 and 9 µg/mL, where the protein component of 25 kDa showed biological activity and for the doses of 18 µg/mL no significant difference was found, since both samples were able to inhibit 100 % of the tumors cells.

These results evidenced that the protein component of 25 kDa has a higher capacity to inhibit the tumors cells growth, than the protein component of 50 kDa and that both components showed biological activity in vitro in an independent manner.

This purification schedule was repeated several times with consistent homogeneous results.

### EXAMPLE 7. Composition of Serralysin polypeptide with prodigiosin.

The protein components and the prodigiosin were formulated in one same composition that increased significantly (p<0.005) the inhibitory effect with respect to its independent effect. The Fig. 8 graphic the IC₅₀ of the anti-proliferative biomolecule isolated from MG2327 preparation and its compositions. The MG2327 preparation is referred to total protein. The composition was realized maintaining the same relation of protein and prodigiosin as the ones employed when the independent components were evaluated. In such composition the prodigiosin can be in concentration of 0.1 - 100 nM, and the Serralysin fragments of 0.1 - 150 µg/mL.

### EXAMPLE 8. p50 gender p25.

Anti-p50 antibodies obtained in sheep was utilized to know the relation between p25 and p50. MG2327 was applied to an SDS-PAGE gel al 12 % and with an Imidazol-Zinc staining (Hardy, E., Santana, H., Sosa, A., Hernandez, L., Fernández-Padrón, C. and Castellanos-Serra, L. 1996. Analytical biochemistry. 240:150-152). Approximately the proteins bands of 50 and 25 kDa, (Fig. 9) were cut, renaturalized in gel and applied to SDS-PAGE. These were transferred to nitrocellulose membrane and the Western Blot was realized. The anti-p50 polyclonal antibody recognized to p25 and degradation fragments of p50. The molecular sizes were estimated with pre-staining molecular weigh markers (Bio-Rad). The Western blot showed here is representative of three equals experiment

### EXAMPLE 9. The degradation of p50 products source to temperature are more active that 50 itself.

The obtained p50 in the example 6 was incubated at different temperatures and its anti-proliferative activity was tested in HEp.2cells, using the MTT method already previously described. The degradation pattern produced to each condition (4, 37, 45 and 60 °C) was analyzed by densitometry.

The generation of fragments because of the degradation was directly proportional with the temperature increment, therefore when the amount of p50 decreased, p25 increased the as product of the degradation of p50 (Fig. 10). The products of the degradation of p50 showed higher anti-proliferative activity than the original p50 (Fig. 11).

### EXAMPLE 10. p25 induces regression of malignant tumors.

The protein p25 obtained by the chromatography described in the Example 6, was applied to reverse phase chromatography of (RP-HPLC), to verify its homogeneousness and purity. A gradient of acetonitrile of 0-100 -in 100 min was used. A peak of proteins with purity major of 90% was observed, which demonstrated the homogeneity of the purified eluate.

The p25 was then injected i.p. to mice BALB/c after of 8 days of implantation of the P3X63Ag8 myeloid tumor and perfectly developed. The doses of 22 µg/kg of weigh of p25 induced total regression in the 80% of the treated animals. The negative controls died at the end of 30 days, wherein already had developed solid tumors.

EXAMPLE 11. p50 is a metalloprotease, while p25 has no proteolytic activity. The modified method of Anson and Mirsky (Anson, M.L., Mirsky, A.E. 1932. J. Gen. Physiol. 16: 59) using casein as a substrate, was adjusted in our laboratory with Trypsine (y=1.9314x-0.682; R²=0.999). The protein fractions obtained in the chromatography describe in the Example 6 were assayed with this method. p50 showed proteolytic activity that was inhibited with 7 mM de EDTA, which turned out to be a metalloprotease. p25 showed no enzymatic activity, Fig. 12.

The method of gel zymography using gelatin as substrate (Vacca, A., lurlaro, M., Ribatti, D., Minischetti, M., Nico, B., Ria, R., Pellegrino, A. and Dammacco, F. 1999. Blood. 94:4143-4155) was employed to verify the proteolytic activity of p50 and p25. Moreover the enzymatic capacity of the p50 degradation was analyzed. In this assay the protein p50 obtained by the chromatography describe in the Example 6 showed enzymatic activity. The protein band fraction of the gel to the height of 25 kDa (from MG2327) showed proteolytic activity; while the p25 obtained by chromatographydid not show this proteolytic activity.

### EXAMPLE 12. Identification of anti-proliferative polypeptides of 25 kDa from MG2327.

To the identification of proteins with anti-proliferative activity present in the band of 25 kDa, the SDS-PAGE gel was cleaved (described in the Example 8) using MG2327. The band was incubated during 5 min in 1 mL of Tris/HCl (100 mM pH 8.5) buffer until this band was totally translucent. The band was cleaved in small cube of nearly 1 mm³, absorbing with acetonitrile, rehydrated in a smallest volume of ammonium bicarbonate (25 mM) containing trypsin or LEP to a concentration of 12.5 ng/µL. The digestion in gel was incubated at 37°C by 18 h in a thermostatic mixer.

The resultants peptides of the LEP digestion were analyzed by MALDI-MS. The monoisotopic ions of the most intense signals were introduced in the ProFound program for the identification of the protein of interest in the sequence data base. Although we did not performed taxonomic restriction during the search in the data base, the 50 kDa protease of *Serratia marcescens* EC 3.4.24.40 was ranked as having the highest similarity. Four peptides (51-57, 58-66, 67-80 and 81-90) belong to the N-terminal region and one (402-409) belonging to C-terminal region of the protein. The molecular size EC 3.4.24.40 differs from the ones present in the analyzed band (nearly 25 kDa, estimated by SDS-PAGE). This finding suggest that the band of 25 kDa contains two 25 kDa fragments that co-migrate with similarity to the 50 kDa protein EC 3.4.24.40 belonging to the Serralysin family.

To confirm this hypothesis a tryptic digestion of the 25 KDa band was developed. The ESI-MS spectrum of the removed peptides was de-convoluted and the most intensive signals were introduced in the Profound program. The output showed the same protein previously identified (EC 3.4.24.40).The sequence cover of the tryptic digestion (21 %) was higher than before digestion (10 %). The sequence cover map proved seven peptides that coincided very well with some of the tryptic fragments PRZN_SERMAlPRZN_SERSP of the proteins. Five of them (28-41, 58-66, 67-80, 81-90 and 163-171) corresponded to the N-terminal region, while that the remainder two peptides (351-373 and 374-393) correspond to the C-terminal region. These results not only confirmed the prior identification of the protein, but also suggested that the map cover the presence of two co migrating fragments of 25 kDa of the identified protein as PRZN_SERMA/PRZN_SERSP, in the analyzed band The ESI-MS/MS spectrum corresponding to peptides of the N- and C-terminal regions of the previously mentioned proteins was manually interpreted and the partials sequences were extracted to its identification,Table 3.

**Table 3. Manual interpretation of the ESI-MS/MS spectrum of five peptides presents in the 25 kDa band. The peptides from 1 belong to the N-terminal region of the PRZN_SERMA/PRZN_SERSP proteins, while the peptide -5 corresponds to the- C-terminal region of these same proteins.**

| *m₁* | *Sequence Tag* | *m₂* | *Peptide sequence* | *z* | *m*/*z expected* | *m*/*z theoretical* | *Error* |
|---|---|---|---|---|---|---|---|
| 705.47 | AQENS | 1235.74 | 28-41 | 2 | 792.91 | 792.89 | 0.02 |
| 522.28 | TFSF | 1004.54 | 58-66 | 2 | 559.29 | 559.28 | 0.01 |
| 677.38 | AVN | 961.58 | 67-80 | 2 | 692.35 | 692.34 | 0.01 |
| 730.40 | EAS | 1017.58 | 81-90 | 2 | 582.79 | 582.79 | 0.00 |
| 1117.84 | GGFX | 1493.08 | 351-373 | 2 | 1031.45 | 1031.48 | 0.03 |

With the employed methods and the obtained results we concluded that in the 25 kDa analyzed band one mixture of proteins exists that has fragments with similarity to the N and C-terminus of the PRZN_SERMA/PRZN_SERSP proteins.

### EXAMPLE 13. Identification of the p50 protein.

To identify the protein p50 with anti-proliferative activity, the protein fraction corresponding to the 50 kDa protein, obtained from the protocol of purification described in Example 6, was digested with Lys-C endoproteinase. The identification of the peptides was performed by means of sequence by automatic Edman Degradation and a JMS HX- 110 double sector masses spectrometer, with FAB cannon. From the obtained results and the alignments realized by the Swissprot and PIR software it was concluded that such protein belongs to the Serralysins family, with 50 kDa molecular weight. The highest similarity was found for the species with the PRZN_SERSP and PRZN_SERMA identification in the Swissprot bank. The molecular mass of all analyzed peptides by mass spectrometer coincided with the expected theoretic amount of peptides of these digesting proteins with Lys-C endoproteinase.

### EXAMPLE 14. Identification of the purified p25 by chromatography.

To identify the 25 kDa protein with anti-proliferative, apoptotic and anti-angiogenic activity, the purified p25 by DEAE chromatography described in the Example 6 was applied to SDS-PAGE. The protein band was washed during five minutes with 500 µL of water, distained with a citric acid solution 100 mM, subsequently was newly washed with MilliQ water, cut in small cubes of approximately 1 mm³. After, acetonitrile was added until its dehydrating and its excess was eliminated. The gel cubes were totally dehydrated in an evaporative centrifuge and subsequently rehydrated in a ammonium bicarbonate solution (50 mM) that contained trypsin to a concentration of 12.5 ng/µL. After that incubated in a thermostatic centrifuge during 30 minutes to 37°C overnight

The peptides were passively eluted adding 20 µL of ammonium bicarbonate solution and additionally incubated at 37°C during 45 minutes. The peptides were extracted using the ZipTip_{c18}^{™} and subsequently the mixture was acidified adding to the reaction 5 µL of pure formic acid, and the peptides were newly extracted using the ZipTp_{c18}^{™}. The adhered peptides to the ZipTip_{c18}^{™} were repeatedly washed with a 5% formic acid solution and subsequently eluted in a volume of 2-3 µL of a 60% acetonitrile solution containing 1% of formic acid.

The originated peptide during the digestion were loaded in borosilicate capillary needles gold covered and introduced in the ionization fountain of the orthogonal geometric hybrid mass spectrometer equipped with a (QTOF-2^{™}) nanospray fountain.

ESI-MS mass spectrum was acquired in a range 350-2000 Da during 1 second. The most intense signals were selected to its posterior ESI-MSMS sequence. The employee collision gas was the argon and it used collision energy appropriate to produce an extensive fragmentation of the selected peptides, which will allow its unequivocal identification in the databases.

The ESI-MS spectrum were deconvoluted, exported in a DTA format and import in the MASCOT program to the identification of the protein in the SWISSPROT and NCBInr databases using the Peptide Mass Fingerprint (PMF) strategy. To an exact identification of the deconvoluted protein, an internal calibration was used using a trypsin autophotolytic peptide fixing an error of 0.05 Da to made the search of the peptides observed in the spectrum and the signals that had an greater intensity to 10% of the intensity of the base peak were selected.

Four peptides presents in the analyzed band were sequenced by ESI-MSMS (Table 4). The peptides PRZN-SERMA/PRZN-SERSP belong to the C-terminal region (indicate in red in the sequence of Table 6), previously identified in the anterior Examples.

**Table 4. Peptides belong to the p25 de S. marcescens that were sequenced by ESI-MSMS.**

| # | *Amino acid sequence* | *mlz tear.* | *m*/*z exp* | *Error* | *SEQ lD NO* |
|---|---|---|---|---|---|
| *1* | **DFLSTTSNSQK** | **1226-51** | **1226.58** | 0.07 | SEQ ID NO: 10 |
| *2* | **SAASDSAPGASDWIR** | **1489.75** | **1489.68** | 0.07 | SEQ lD NO: 11 |
| *3* | **GGAGNDVLFGGGGADELWGGAGK** | **2060.96** | **2060.87** | 0.11 | SEQ ID NO: 12 |
| *4* | **TGDTVYGFSNTGR** | **1488.65** | **1488.60** | 0.05 | SEQ ID NO: 13 |

Likewise others signals were founded that were not sequenced, it's mass values concord very good with expected mass values for tryptic peptides identified as PRZN_SERMA/PRZN_SERSP of the C-terminal region of the proteins, Table 5 (noted in blue in the Table 6). Among these peptides, appears a double charged signal that could correspond with the C-terminal peptide of the PRZN_SERMA/PRZN_SERSP proteins. No peptides were founded that could be assigned to specific cuts of the PRZN_SERMA/PRZN_SERSP proteins N-terminal region.

**Table 5. Peptides tryptics belonging to the PRZN_SERMA protein detected in the ESI-MS spectrum.**

| *#* | *Amino acid Sequence* | *m*/*z exp.* | *m*/*z calc.* | *z* | *error* | *SEQ ID NO* |
|---|---|---|---|---|---|---|
| *1* | **³²⁵SFSDVGGLK³¹³** | **455.20** | **455.24** | 2 | 0.04 | SEQ ID NO: 14 |
| *2* | **⁴¹⁷IDLSFFNK⁴²⁴** | **492.24** | **492.26** | 2 | 0.02 | SEQ ID NO: 15 |
| *3* | **⁴⁷⁵IVGQVDVATDFIV⁴⁸⁷** | **688.35** | **688.38** | 2 | 0.03 | SEQ ID NO: 16 |

The exhibited methods and results in this example were able to ensure that in the 25 kDa band, obtained from the p25 purified by DEAE chromatography, and highly anti-proliferative, a fragment of the C-terminal 25 kDa PRZN_SERMA/PRZN_SERSP proteins is present.

Table 6. Identification of the p50 and p25 obtained by DEAE chromatography. In the sequence, the amino_acids that are not presents in the mature protein are crossed. Without these amino acids the molecular weights of these proteins are 50293.4 Da and 50595.4 Da for PRZN_SERSP and PRZN_SERMA respectively. The identification of the tryptics peptides that difefer between both molecules suggest that both species may be present and even coexist (green (*italic*): identified by mass spectrometry and maroon (emphasized): identified by Edman degradation inside the continuous rectangle). The peptides marked and were identified in the p25 obtained by chromatography described in the Example 6. The peptides marked and underlined were identified in the p50 obtained by the chromatography described in Example 6 and the peptide in (*italic*) was identified from a gel fragment.

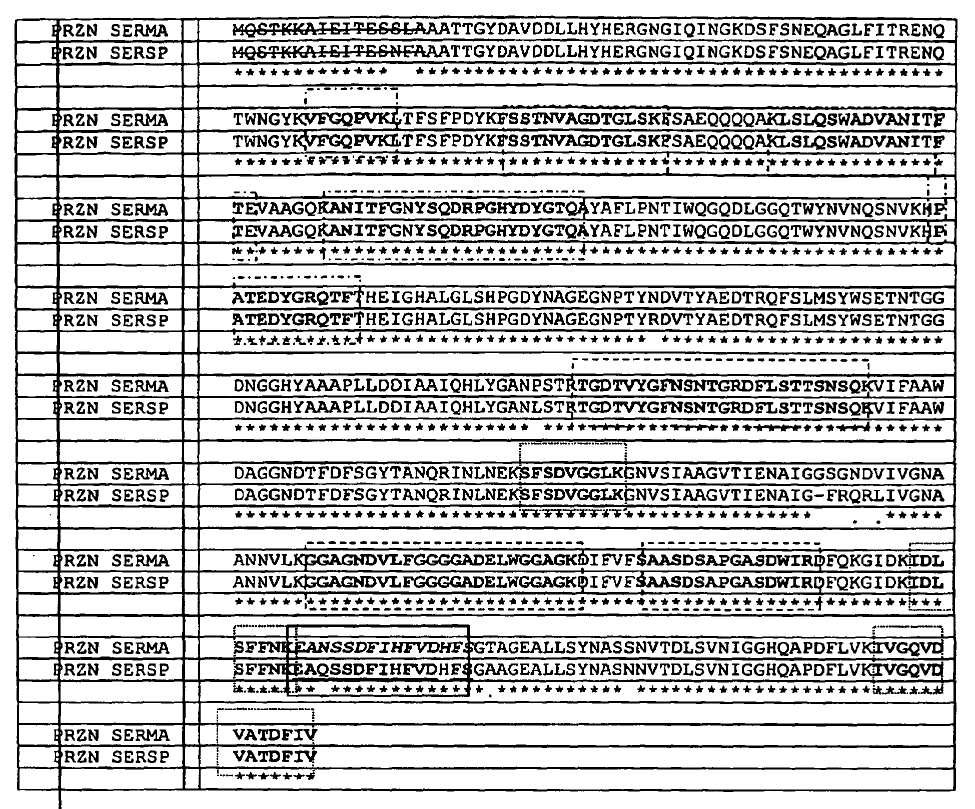

### EXAMPLE 15. Proteins of the N- and C- terminal with 25 kDa.

To determine the molecular size of the proteins that could coexist at the level of 25 kDa in SDS-PAGE, originated from degradations of . PRZN_SERMA/PRZN_SER, their sequence were introduced in the GenRun program. The fragments corresponding to the N- and C-terminus of 25 kDa (± 2 kDa) are shown in Table 7.

**Table 7. Proteins with size of 25 ± 2 kDa, obtained from degradation of the PRZN_SERMA/PRZN_SERSP. The molecular weight was determined using the GenRun program, from the N- and C-terminal ends.**

| *Protein* | *Sequence* | *SEQ ID NO* |
|---|---|---|
| ARA1 | | SEQ ID NO: 1 |
| ARA2 | | SEQ ID NO: 2 |
| ARA3 | | SEQ ID NO: 3 |
| ARA4 | | SEQ ID NO: 4 |

### EXAMPLE 16. MG2327- induced apoptosis.

### MG2327 induces apoptosis on myeloma X63 cells, with DNA fragmentation and involving mitochondria and microtubules.

To determine the kind of tumoral cell death experience by tumoral cells, murine myeloma P3X63Ag8 cells (2*10⁷) were treated *in vitro* with MG2327 at 22 µg/ml. Treated and untreated cells were prepared for ultrastructural transmission electron microscopy studies at different times, and genomic DNA was extracted for DNA laddering assay.

DNA fragmentation was assessed in 2% agarose gel electrophoresis. Apoptosis frequently involves cellular DNA laddering of 180-200 bp, representative of inter-nucleosomal distances (Soldatenkov, V. A., Prasad, S., Voloshin, Y., and Dritschilo, A. 1998. Cell Death Differ. 5:307-12). As shown in figure 13, a significant increase of oligonucleosomes formation was observed, that were correlated to the morphological changes observed in culture and by electron microscopy.

Inter-nucleosomal fragmentation was preceded by morphological signs of apoptosis detected by optical microscopy. Moreover, micrographs showed altered cytoplasmatic organelles (mitochondrion, 2h), also preceding chromatin condensation (4h) and internucleosomal fragmentation (6h).

### MG2327 affects microtubules and the ultrastructural organization of mitochondria, increasing P3X63Ag8 cells apoptosis.

Untreated cells showed a typical mitochondrial ultrastructure: clearly visible mitochondrial cristae and a mitochondrial matrix of higher density, evenly distributed all over the cytoplasm (Fig. 14A).

Cells treated with MG2327 showed increased mitochondria size, a lower matrix density and heavily affected cristae morphology (Fig. 14B-E). These ultrastructural changes mostly indicate dysfunctional organelles.

Moreover, we observed extensive cytoplasmatics vacuoles, also regarding the endoplasmic reticulum as mitochondria and nuclear structure, 2 hrs after treatment (Fig. 14B).

Different morphological changes were observed in the nuclei at 6 hr treatment (chromatin condensing, merging and laddering). In the late phase apoptosis micrograph (8 hr) of P3X63Ag8 cells treated with MG2327, chromatin appeared compact (Fig. 14F). Budding was not detected at any time.

Interestingly, mitochondria in P3X63Ag8 cells treated for 2 hr were clustered at cytoplasmic membrane periphery (Fig. 14B-E), resulting from microtubule disruption and interfered mitochondria transport along this organelle. (Schatten, H., and Lewis, M.L. 2001. Acta Astronaut. 49:399-418).

Figures 14C and D show greater mitochondria, also bearing condensed structures attached to the inner mitochondrial membranes. These structures would have been generated by successive cristae fusion. MG2327 could generate apoptosis by cytochrome-C released into the cytosol after mitochondrial outer membrane swelling while increasing in size, followed by caspase activation and apoptosis (Green, D.R. and Reed, J.C. 1998. Science 28:1309-1312. Review).

Without increasing in size, cytochrome c could also be completely and quickly released from damaged cristae of the mitochondrion to the cytosol, through junctions between the intermembrane space and cristae after fusion (Scorrano, L.., Ashiya, M., Buttle, K., Weiler, S., Oakes, S.A., Mannella, C.A. and Korsmeyer, S.J. 2002. Dev Cell. 2:55-67). The process described significantly amplified apoptosis signals generated by MG2327 into cells.

### Example 17. p25 and p50 proteins induce apoptosis.

To know if p25 and p50 proteins could be involved in the apoptotic events induced by MG2327, they were individually administered to P3X63Ag8 cells at different concentrations, and analyzed by electron microscopy. In all cases, chromatin condensing, damaged mitochondria cristae and clustered mitochondria were detected. Indeed, apoptosis induction by MG2327 is linked to the effect of these two proteins.

### Example 18. Antiangiogenic effect of MG2327 and the anti-proliferating polypeptides.

### Development of tubular structures in matrigel

Human micro-vasculature-derived human endothelial cells (HMEC) were assessed for endothelial cord formation on matrigel (Crum R, Szabo S, Folkman J. 1985. Science. 230:1375-8; Vacca, A., Ribatti, D., Presta, M., Minischettti, M., lurlaro, M, Ria, R, Albini, A, Bussolino, F. and Dammaacco, F. 1999. Blood 93:3064) after culture under non-cytotoxic MG2327, p25 and p50 concentrations (Sanz, L., Pascual, M., Muñoz, A., González, M.A., Salvador CH, Álvarez-Vallina L. 2002. Microvascular Research 63:335-339). Results considered the length of tubular structures and the number of interconnections between them, as calculated by using the Image-Pro Express 4.5 package. They indicated a significant inhibition (p<0.05, ANOVA) of differentiation or maturation of endothelial cells (Fig.15) after treatment with the MG2327 preparation and its p25 and p50 fractions.

### Example 19. Indirect effect of MG2327 on proliferating cells.

### MG2327 protects Balb/c mice from myeloid tumor implants.

To analyze the protective activity of MG2327 against implanted tumors, Balb/c mice were inoculated (i.p.) with 1 mg/ml of this anti-tumoral preparation under different immunization schedules. Three doses were administered, one dose every week, and two doses every week during three weeks with at least three days between doses. A negative control group was inoculated with 1X PBS. Two millions of P3X63Ag8 myeloma cells were inoculated i.p. to the experimental groups (treated and control) 150 days after the first dose (5 months after). All mice from the negative control group died during the first 25 days, while 100% of the treated animals survived without tumors. (Fig. 16).

### EXAMPLE 20 The C-terminal domain of other Serralysins has also cytotoxic effect, without proteolytic activity.

The ATCC14756 strain was cultivated under similar conditions as the CMIB4202 strain according to Example 2 and its culture supernatants were processed as described in Example 6. In both preparations we observed proteins at the levels of 50 kDa that eluted with 50 mM phosphate-0.2 M NaCl, pH 8.00. These proteins showed enzymatic activity that was inhibited with 10 mM of EDTA and recovered with 5 µM Zn₂SO₄. Both proteins were digested chemically with CNBr and the digestion pattern was similar, generating similar fragments of approximately 25 kDa, corresponding to the C-terminus of p50, from the internal methionine in the sequence to the end of the molecule.

The fragments obtained from the digestion were renatured with a change in buffer through dialysis for 48 hours. The biological activity of these fragments was tested in the cytotoxicity assay described here, using HEp.2 cells that were incubated for 72 hours in their presence. The fragments produced by the digestion, and lacking proteolytic activity, in the presence of 5 mM EDTA, presented a higher cytotoxic activity that was dose-dependent, approximately 2.5 times over that of the complete p50 molecule. These fragments, once their sequences are known, can also be obtained by chemical synthesis or by recombination techniques.

### EXAMPLE 21 Combination of fragments of Serralysins with antibodies or antibody fragments.

The polypeptide fragments obtained in Examples 6 and 20 were chemically conjugated with the monoclonal antibody CB/ior-CEA.1 (Tormo B et al. APMIS 97:1073-1080,1989), with its variable regions, and with the antibody fragment obtained via recombinant DNA technology from its sequence (diabody) (patent WO 03/093315). The conjugated biomolecules were assayed on the human tumor cell lines LoVo (ATCC CCL-229), AsPC-1 (ATCC CRL-1682) and LS 174T (ATCC CL-188), all expressing CEA in culture, through an anti-proliferation assays similar to the one described in Example 3. The conjugated fragments were used at cytotoxic concentrations equivalent to those of un-conjugated fragments, with a dose-dependent response, while with the un-conjugated molecules no anti-proliferation response was observed. It was shown that the conjugated fragments were bound to CEA on the cells, using procedures as cell-ELISA, and indirect immunofluorescence (patent WO 03/093315). These results demonstrate that the conjugates described here can be used for the therapy and diagnosis of cancer.

### SEQUENCE LISTING.

<110> CENTRO DE INGENIERIA GENETICA Y BIOTECNOLOGIA
   <120> PHARMACEUTICAL COMPOSITION THAT CONTAINIG POLIPEPTIDE FRAGMENTS OF SERRALISINS
<130> Maria del Carmen
<140>
   <141>
<160> 20
<170> PatentIn Ver. 2.1
<210> 1
   <211> 258
   <212> PRT
   <213> Serratia marcescens
<220>
   <221> PEPTIDE
   <222> (1)..(258)
   <223> ARA1Polipeptide: antiproliferative, apoptotic, antiangiogenic and protector against diseases to be relate with the cellular proliferation and the angiogenic. Sequency of the C-terminal of the SERMA. Serralisin
<400> 1
   Met Ser Tyr Trp Ser Glu Thr Asn Thr Gly Gly Asp Asn Gly Gly His
    1               5                  10                  15
   Tyr Ala Ala Ala Pro Leu Leu Asp Asp Ile Ala Ala Ile Gln His Leu
               20                  25                  30
   Tyr Gly Ala Asn Pro Ser Thr Arg Thr Gly Asp Thr Val Tyr Gly Phe
           35                  40                  45
   Asn Ser Asn Thr Gly Arg Asp Phe Leu Ser Thr Thr Ser Asn Ser Gln
       50                  55                  60
   Lys Val Ile Phe Ala Ala Trp Asp Ala Gly Gly Asn Asp Thr Phe Asp
   65                  70                  75                  80
   Phe Ser Gly Tyr Thr Ala Asn Gln Arg Ile Asn Leu Asn Glu Lys Ser
                   85                  90                  95
   Phe Ser Asp Val Gly Gly Leu Lys Gly Asn Val Ser Ile Ala Ala Gly
               100                 105                 110
   Val Thr Ile Glu Asn Ala Ile Gly Gly Ser Gly Asn Asp Val Ile Val
           115                 120                 125
   Gly Asn Ala Ala Asn Asn Val Leu Lys Gly Gly Ala Gly Asn Asp Val
       130                 135                 140
   Leu Phe Gly Gly Gly Gly Ala Asp Glu Leu Trp Gly Gly Ala Gly Lys
   145                 150                 155                 160
   Asp Ile Phe Val Phe Ser Ala Ala Ser Asp Ser Ala Pro Gly Ala Ser
                   165                 170                 175
   Asp Trp Ile Arg Asp Phe Gln Lys Gly Ile Asp Lys Ile Asp Leu Ser
               180                 185                 190
   Phe Phe Asn Lys Glu Ala Asn Ser Ser Asp Phe Ile His Phe Val Asp
           195                 200                 205
   His Phe Ser Gly Thr Ala Gly Glu Ala Leu Leu Ser Tyr Asn Ala Ser
       210                 215                 220
   Ser Asn Val Thr Asp Leu Ser Val Asn Ile Gly Gly His Gln Ala Pro
   225                 230                 235                 240
   Asp Phe Leu Val Lys Ile Val Gly Gln Val Asp Val Ala Thr Asp Phe
                   245                 250                 255
   Ile Val
<210> 2
   <211> 257
   <212> PRT
   <213> Serratia marcescens
<220>
   <221> PEPTIDE
   <222> (1)..(257)
   <223> ARA2 Polipeptide: antiproliferative, apoptotic, antiangiogenic and protector against diseases to be related with the cellular proliferation and the angiogenic. C-terminal Sequency of the SERSP. Serralisin
<400> 2
   Met Ser Tyr Trp Ser Glu Thr Asn Thr Gly Gly Asp Asn Gly Gly His
   1               5                   10                  15
   Tyr Ala Ala Ala Pro Leu Leu Asp Asp Ile Ala Ala Ile Gln His Leu
               20                  25                  30
   Tyr Gly Ala Asn Leu Ser Thr Arg Thr Gly Asp Thr Val Tyr Gly Phe
           35                  40                  45
   Asn Ser Asn Thr Gly Arg Asp Phe Leu Ser Thr Thr Ser Asn Ser Gln
       50                  55                  60
   Lys Val Ile Phe Ala Ala Trp Asp Ala Gly Gly Asn Asp Thr Phe Asp
   65                  70                  75                  80
   Phe Ser Gly Tyr Thr Ala Asn Gln Arg Ile Asn Leu Asn Glu Lys Ser
                   85                  90                  95
   Phe Ser Asp Val Gly Gly Leu Lys Gly Asn Val Ser Ile Ala Ala Gly
               100                 105                 110
   Val Thr Ile Glu Asn Ala Ile Gly Phe Arg Gln Arg Leu Ile Val Gly
           115                 120                 125
   Asn Ala Ala Asn Asn Val Leu Lys Gly Gly Ala Gly Asn Asp Val Leu
       130                 135                 140
   Phe Gly Gly Gly Gly Ala Asp Glu Leu Trp Gly Gly Ala Gly Lys Asp
   145                 150                 155                 160
   Ile Phe Val Phe Ser Ala Ala Ser Asp Ser Ala Pro Gly Ala Ser Asp
                   165                 170                 175
   Trp Ile Arg Asp Phe Gln Lys Gly Ile Asp Lys Ile Asp Leu Ser Phe
               180                 185                 190
   Phe Asn Lys Glu Ala Gln Ser Ser Asp Phe Ile His Phe Val Asp His
           195                 200                 205
   Phe Ser Gly Ala Ala Gly Glu Ala Leu Leu Ser Tyr Asn Ala Ser Asn
       210                 215                 220
   Asn Val Thr Asp Leu Ser Val Asn Ile Gly Gly His Gln Ala Pro Asp
   225                 230                 235                 240
   Phe Leu Val Lys Ile Val Gly Gln Val Asp Val Ala Thr Asp Phe Ile
                   245                 250                 255
   Val
<210> 3
   <211> 219
   <212> PRT
   <213> Serratia marcescens
<220>
   <221> PEPTIDE
   <222> (1)..(219)
   <223>: ARA3 Polipeptide: antiproliferative, apoptotic, antiangiogenic and protector against diseases to be related with the cellular proliferation and the angiogenic. C-terminal Sequency of the SERSP.Serralisin
<400> 3
   Thr Arg Thr Gly Asp Thr Val Tyr Gly Phe Asn Ser Asn Thr Gly Arg
   1               5                   10                  15
   Asp Phe Leu Ser Thr Thr Ser Asn Ser Gln Lys Val Ile Phe Ala Ala
               20                  25                  30
   Trp Asp Ala Gly Gly Asn Asp Thr Phe Asp Phe Ser Gly Tyr Thr Ala
           35                  40                  45
   Asn Gln Arg Ile Asn Leu Asn Glu Lys Ser Phe Ser Asp Val Gly Gly
       50                  55                  60
   Leu Lys Gly Asn Val Ser Ile Ala Ala Gly Val Thr Ile Glu Asn Ala
   65                  70                  75                  80
   Ile Gly Phe Arg Gln Arg Leu Ile Val Gly Asn Ala Ala Asn Asn Val
                   85                  90                  95
   Leu Lys Gly Gly Ala Gly Asn Asp Val Leu Phe Gly Gly Gly Gly Ala
               100                 105                 110
   Asp Glu Leu Trp Gly Gly Ala Gly Lys Asp Ile Phe Val Phe Ser Ala
           115                 120                 125
   Ala Ser Asp Ser Ala Pro Gly Ala Ser Asp Trp Ile Arg Asp Phe Gln
       130                 135                 140
   Lys Gly Ile Asp Lys Ile Asp Leu Ser Phe Phe Asn Lys Glu Ala Gln
   145                 150                 155                 160
   Ser Ser Asp Phe Ile His Phe Val Asp His Phe Ser Gly Ala Ala Gly
                   165                 170                 175
   Glu Ala Leu Leu Ser Tyr Asn Ala Ser Asn Asn Val Thr Asp Leu Ser
               180                 185                 190
   Val Asn Ile Gly Gly His Gln Ala Pro Asp Phe Leu Val Lys Ile Val
           195                 200                 205
   Gly Gln Val Asp Val Ala Thr Asp Phe Ile Val
       210                 215
<210> 4
   <211> 220
   <212> PRT
   <213> Serratia marcescens
<220>
   <221> PEPTIDE
   <222> (1)..(220)
   <223> ARA4 Polipeptide: antiproliferative, apoptotic, antiangiogenic and protector against diseases to be related with the cellular proliferation and the angiogenic. C-terminal Sequency of the SERMA. .Serralisin
<400> 4
   Thr Arg Thr Gly Asp Thr Val Tyr Gly Phe Asn Ser Asn Thr Gly Arg
    1               5                  10                  15
   Asp Phe Leu Ser Thr Thr Ser Asn Ser Gln Lys Val Ile Phe Ala Ala
               20                  25                  30
   Trp Asp Ala Gly Gly Asn Asp Thr Phe Asp Phe Ser Gly Tyr Thr Ala
           35                  40                  45
   Asn Gln Arg Ile Asn Leu Asn Glu Lys Ser Phe Ser Asp Val Gly Gly
       50                  55                  60
   Leu Lys Gly Asn Val Ser Ile Ala Ala Gly Val Thr Ile Glu Asn Ala
   65                  70                  75                  80
   Ile Gly Gly Ser Gly Asn Asp Val Ile Val Gly Asn Ala Ala Asn Asn
                   85                  90                  95
   Val Leu Lys Gly Gly Ala Gly Asn Asp Val Leu Phe Gly Gly Gly Gly
               100                 105                 110
   Ala Asp Glu Leu Trp Gly Gly Ala Gly Lys Asp Ile Phe Val Phe Ser
           115                 120                 125
   Ala Ala Ser Asp Ser Ala Pro Gly Ala Ser Asp Trp Ile Arg Asp Phe
       130                 135                 140
   Gln Lys Gly Ile Asp Lys Ile Asp Leu Ser Phe Phe Asn Lys Glu Ala
   145                 150                 155                 160
   Asn Ser Ser Asp Phe Ile His Phe Val Asp His Phe Ser Gly Thr Ala
                   165                 170                 175
   Gly Glu Ala Leu Leu Ser Tyr Asn Ala Ser Ser Asn Val Thr Asp Leu
               180                 185                 190
   Ser Val Asn Ile Gly Gly His Gln Ala Pro Asp Phe Leu Val Lys Ile
           195                 200                 205
   Val Gly Gln Val Asp Val Ala Thr Asp Phe Ile Val
       210                 215                 220
<210> 5
   <211> 5
   <212> PRT
   <213> Serratia marcescens
<220>
   <221> PEPTIDE
   <222> (1)..(5)
   <223> Serralisins Sequency
<400> 5
   Ala Gln Glu Asn Ser
   1 5
<210> 6
   <211> 4
   <212> PRT
   <213> Serratia marcescens
<220>
   <221> PEPTIDE
   <222> (1)..(4)
   <223> Serralisins Sequency
<400> 6
   Thr Phe Ser Phe
   1
<210> 7
   <211> 3
   <212> PRT
   <213> Serratia marcescens

## Claims

1. A pharmaceutical composition **characterized by** containing one or several Serralysin fragments having the amino acid sequence selected from the group consisting of SEQ. ID. No. 1, SEQ. ID. No. 2, SEQ. ID. No. 3, SEQ. ID. No. 4, SEQ ID NO. 10, SEQ ID NO. 11, SEQ ID NO. 12, SEQ ID NO. 13, SEQ ID NO. 14, SEQ ID NO. 15, and SEQ ID NO. 16, for use in inducing antitumoral effects in the recipient organism, and said antitumoral effects are therapeutic or preventive.

2. A pharmaceutical composition for use according to claim 1, wherein said Serralysin fragments are obtained from cell culture supernatants, by genetic manipulation or by chemical synthesis.

3. A composition for use according to claims 1 and 2, wherein said fragments appear in the composition alone, conjugated or mixed.

4. A composition for use according to any one of claims 1-3, wherein said Serralysin fragments are part of chimeric or hybrid molecules obtained by genetic manipulation or chemical synthesis.

5. Serralysin fragments selected from the group consisting of SEQ. ID. No. 1-4 and SEQ. ID. No. 10-16 for use in the treatment of proliferative diseases.

6. Serralysin fragments for use according to claim 5, wherein said fragments are part of a composition containing antibodies, antibody fragments, nucleic acid strands, or carbohydrate molecules or protein molecules, and said fragments are alone, conjugated, or mixed.

7. Serralysin fragments for use according to claim 5 or 6, alone or in combination, **characterized by** being part of said composition as molecular aggregates, alone, in combination between them, or in combination with other protein or carbohydrate molecules.

8. An in vitro diagnostic method for pathologies related to the overexpression of receptors for growth factors and inflammatory diseases comprising the use of Serralysin fragments according to claims 5 to 7, alone or in combination.

9. A screening method for pathologies related to the overexpression of receptors for growth factors and inflammatory diseases comprising use of Serralysin fragments according to any one of claims 5 to 7, alone or in combination.

10. A composition for use according to any one of claims 1 to 4, **characterized by** further containing one or several prodigiosins, wherein said prodigiosins enhance the antitumoral activity of the mentioned composition.

11. A composition for use according to any one of claims 1 to 4 and 10 for use in the treatment of pathological conditions related with angiogenesis, proliferating cells and immune system.

12. A composition for use according to any one of claims 1 to 4 and 10 for use in the treatment of pathological conditions that can be treated by induction of anti-proliferating, apoptotic, anti-angiogenic, immunomodulating or differentiation-regulating factors.

## Patentansprüche

1. Pharmazeutische Zusammensetzung, **dadurch gekennzeichnet, dass** sie ein oder mehrere Serralysin-Fragmente mit einer Aminosäuresequenz, die aus der Gruppe bestehend aus SEQ ID NO 1, SEQ ID NO 2, SEQ ID NO 3, SEQ ID NO 4, SEQ ID NO 10, SEQ ID NO 11, SEQ ID NO 12, SEQ ID NO 13, SEQ ID NO 14, SEQ ID NO 15 und SEQ ID NO 16 ausgewählt ist, enthält, zur Verwendung beim Induzieren antitumoraler Wirkungen im Empfängerorganismus, wobei die besagten antitumoralen Wirkungen therapeutisch oder präventiv sind.

2. Pharmazeutische Zusammensetzung zur Verwendung gemäß Anspruch 1, wobei die besagten Serralysin-Fragmente aus Zellkulturüberständen, durch genetische Manipulation oder durch chemische Synthese gewonnen werden.

3. Zusammensetzung zur Verwendung gemäß den Ansprüchen 1 und 2, wobei die besagten Fragmente in der Zusammensetzung allein, konjugiert oder gemischt vorkommen.

4. Zusammensetzung zur Verwendung gemäß einem der Ansprüche 1-3, wobei die besagten Serralysin-Fragmente Teil von chimären oder Hybridmolekülen, die mittels genetischer Manipulation oder chemischer Synthese gewonnen werden, sind.

5. Serralysin-Fragmente, ausgewählt aus der Gruppe bestehend aus SEQ ID NO 1-4 und SEQ ID NO 10-16 zur Verwendung in der Behandlung von proliferativen Erkrankungen.

6. Serralysin-Fragmente zur Verwendung gemäß Anspruch 5, wobei die besagten Fragmente Teil einer Zusammensetzung, die Antikörper, Antikörper-Fragmente, Nukleinsäurestränge oder Kohlenhydratmoleküle oder Proteinmoleküle enthält, sind und die besagten Fragmente allein, konjugiert oder gemischt sind.

7. Serralysin-Fragmente zur Verwendung gemäß Anspruch 5 oder 6, allein oder in Kombination, **dadurch gekennzeichnet, dass** sie Teil der besagten Zusammensetzung als molekulare Aggregate, allein, in Kombination unter sich oder in Kombination mit anderen Protein- oder Kohlenhydratmolekülen sind.

8. Diagnostisches In-vitro-Verfahren für pathologische Zustände, die mit der Überexpression von Rezeptoren für Wachstumsfaktoren und mit inflammatorischen Erkrankungen in Beziehung stehen, welches die Verwendung von Serralysin-Fragmenten gemäß den Ansprüchen 5-7, allein oder in Kombination, umfasst.

9. Screening-Verfahren für pathologische Zustände, die mit der Überexpression von Rezeptoren für Wachstumsfaktoren und mit inflammatorischen Erkrankungen in Beziehung stehen, welches die Verwendung von Serralysin-Fragmenten gemäß einem der Ansprüche 5-7, allein oder in Kombination, umfasst.

10. Zusammensetzung zur Verwendung gemäß einem der Ansprüche 1 bis 4, **dadurch gekennzeichnet, dass** sie weiter ein oder mehrere Prodigiosine enthält, wobei die besagten Prodigiosine die antitumorale Wirksamkeit der erwähnten Zusammensetzung verstärken.

11. Zusammensetzung zur Verwendung gemäß einem der Ansprüche 1 bis 4 und 10 zur Verwendung in der Behandlung pathologischer Zustände, die mit Angiogenese, proliferierenden Zellen und dem Immunsystem in Beziehung stehen.

12. Zusammensetzung zur Verwendung gemäß einem der Ansprüche 1 bis 4 und 10 zur Verwendung in der Behandlung pathologischer Zustände, die mit der Induktion von antiproliferativen, apoptotischen, antiangiogenen, immunmodulierenden oder die Differenzierung regulierenden Faktoren behandelt werden können.

## Revendications

1. Composition pharmaceutique **caractérisée en ce qu'**elle contient un ou plusieurs fragments de Serralysine ayant la séquence d'acides aminés choisie parmi le groupe consistant en SEQ ID No. 1, SEQ ID No. 2, SEQ ID. No. 3, SEQ ID No. 4, SEQ ID NO. 10, SEQ ID NO. 11, SEQ ID NO. 12, SEQ ID NO. 13, SEQ ID NO. 14, SEQ ID NO. 15 et SEQ ID NO. 16, destinée à une utilisation d'induire des effets antitumoraux dans l'organisme receveur, et lesdits effets antitumoraux sont thérapeutiques ou préventifs.

2. Composition pharmaceutique destinée à une utilisation selon la revendication 1, où lesdits fragments de Serralysine sont obtenus à partir de surnageants de culture cellulaire, par manipulation génétique ou par synthèse chimique.

3. Composition destinée à une utilisation selon les revendications 1 et 2, où lesdits fragments sont présents dans la composition seuls, conjugués ou mélangés.

4. Composition destinée à une utilisation selon l'une quelconque des revendications 1 à 3, où lesdits fragments de Serralysine font partie de molécules chimériques ou hybrides, obtenues par manipulation génétique ou par synthèse chimique.

5. Fragments de Serralysine choisis parmi le groupe consistant en SEQ ID No. 1-4 et SEQ ID No. 10-16, destinés à être utilisés dans le traitement de maladies prolifératives.

6. Fragments de Serralysine destinée à une utilisation selon la revendication 5, où lesdits fragments font partie d'une composition contenant des anticorps, des fragments d'anticorps, des brins d'acide nucléique, ou des molécules de glucides ou des molécules de protéines, et lesdits fragments sont seuls, conjugués ou mélangés.

7. Fragments de Serralysine destinée à une utilisation selon la revendication 5 ou 6, seuls ou en combinaison, **caractérisés en ce qu'**ils font partie de ladite composition sous forme d'agrégats moléculaires, seuls, en combinaison entre eux, ou en combinaison avec d'autres molécules de protéines ou de glucides.

8. Procédé in vitro de diagnostic pour des pathologies associées à la surexpression de récepteurs de facteurs de croissance et des maladies inflammatoires comprenant l'utilisation de fragments de Serralysine selon les revendication 5 à 7, seuls ou en combinaison.

9. Procédé de dépistage de pathologies associées à la surexpression de récepteurs de facteurs de croissance et de maladies inflammatoires comprenant l'utilisation de fragments de Serralysine selon l'une quelconque des revendications 5 à 7, seuls ou en combinaison.

10. Composition destinée à une utilisation selon l'une quelconque des revendications 1 à 4, **caractérisée en outre en ce qu'**elle contient une ou plusieurs prodigiosines, où lesdites prodigiosines augmentent l'activité antitumorale de la composition mentionnée.

11. Composition destinée à une utilisation selon l'une quelconque des revendications 1 à 4 et 10, destinée à être utilisée dans le traitement d'états pathologiques associés à l'angiogenèse, à la prolifération cellulaire et au système immunitaire.

12. Composition destinée à une utilisation selon l'une quelconque des revendications 1 à 4 et 10, destinée à être utilisée dans le traitement d'états pathologiques qui peuvent être traités par l'induction de facteurs d'anti-prolifération, apoptotiques, anti-angiogéniques, d'immunomodulation ou de régulation de la différenciation.
